# EUROPÄISCHE PATENTSCHRIFT

(11) **EP 1 871 250 B1**
(45) Veröffentlichungstag und Bekanntmachung des Hinweises auf die Patenterteilung: **31.12.2014**
(21) Anmeldenummer: 05733144.9
(22) Anmeldetag: 16.04.2005
(51) Int. Cl.: A61B 17/70, A61B 17/00, A61B 17/86

(54) **BANDSCHEIBENENTLASTUNGSIMPLANTAT**
IMPLANT FOR ALLEVIATING PRESSURE ON INTERVERTEBRAL DISKS
IMPLANT DE DELESTAGE DE DISQUE INTERVERTEBRAL

(43) Veröffentlichungstag der Anmeldung: 02.01.2008
(73) Patentinhaber: Aesculap AG, 78532 Tuttlingen (DE)
(72) Erfinder: FINK, Ulrich, 78532 Tuttlingen (DE); WING, Charles, Bethlehem Twp., PA 18020 (US)
(74) Vertreter: Hoeger, Stellrecht & Partner Patentanwälte
(86) Internationale Anmeldenummer: PCT/EP2005/004060
(87) Internationale Veröffentlichungsnummer: WO 2006/111174

(56) Entgegenhaltungen:
- WO-A-2004/073532
- WO-A-2005/025461
- FR-A- 2 816 197
- US-A- 5 645 599
- US-A1- 2004 106 995
- US-A1- 2004 230 309

## Beschreibung

Die vorliegende Erfindung betrifft ein Bandscheibenentlastungsimplantat zum Aufrichten und Entlasten eines Bandscheibenfachs einer menschlichen oder tierischen Wirbelsäule, mit mindestens zwei Anlageelementen für jeweils einen Dornfortsatz zum Anlegen oder Festlegen des Implantats an einem oder zwei Dornfortsätzen benachbarter Wirbel der Wirbelsäule, wobei das Implantat aus einem biokompatiblen, resorbierbaren Material hergestellt ist.

Ferner wird nachfolgend ein Verfahren zum Aufrichten und Entlasten eines Bandscheibenfachs einer menschlichen oder tierischen Wirbelsäule, unter Verwendung eines Bandscheibenentlastungsimplantats mit mindestens zwei Anlageelementen für jeweils einen Dornfortsatz zum Anlegen und/oder Festlegen des Implantats an einem oder zwei Dornfortsätzen benachbarter Wirbel der Wirbelsäule, beschrieben.

Bei einem Prolaps einer zwischen zwei Wirbelkörpern der Wirbelsäule angeordneten Bandscheibe tritt in der Regel Nukleusmaterial durch den Anulus der Bandscheibe aus. Eine Folge hiervon ist ein Verlust der ursprünglichen Band-scheibenhöhe, mit der Folge, daß sich die Knochenstrukturen des Formens annähern und dabei auf Wurzeln von der Wirbelsäule austretender Nervenstrukturen drücken. Aufgrund dieses mechanischen Drucks leidet ein Patient häufig unter sehr starken Schmerzen.

Ist die Hülle des Anulus, das sogenannte Containment, nur lokal begrenzt zerstört, besteht die Möglichkeit, durch Injektion von Nukleus- oder Knorpelzellen in den Nukleus diesen und damit das gesamte Bandscheibenfach wieder aufzurichten. Allerdings ist dabei zu beachten, daß infolge der Injektion eines großen Zellvolumens die gewünschte Höhe zwar sofort restauriert, aber auch ein sehr großer Druck im Innern der Bandscheibe entsteht, der zum Absterben der Zellen führen kann oder sogar fast immer führt. Dieses Problem läßt sich umgehen, indem nur ein geringes Zellvolumen in den Nukleus injiziert wird, sodaß kein erhöhter Druck entsteht. Das Volumen innerhalb der Bandscheibe wird dann erst durch die Bildung der extrazellulären Matrix durch die injizierten Zellen gesteigert. Nachteil dabei ist, daß ein Patient erst mittel- oder langfristig schmerzfrei werden kann und sehr häufig mehrere Eingriffe erforderlich sind.

Vorrichtungen und Verfahren zum Einsetzen von Wirbelsäulenimplantaten sind insbesondere aus der WO 2005/025461 A2 bekannt, die ein Bandscheibenentlastungs-implantat mit den Merkmalen des Oberbegriffs des Anspruchs 1 offenbart. Es ist daher Aufgabe der vorliegenden Erfindung, ein Bandscheibenentlastungsimplantat der eingangs beschriebenen Art so zu verbessern, daß möglichst nur ein einziger Eingriff zum Aufrichten und Entlasten des Bandscheibenfachs erforderlich wird.

Diese Aufgabe wird bei einem Bandscheibenentlastungsimplantat der eingangs beschriebenen Art erfindungsgemäß dadurch gelöst, daß ein Abstand zwischen den mindestens zwei Anlageelementen veränderbar ist und daß die zwei Anlageelemente in einem bestimmten Abstand relativ zueinander festlegbar sind.

Ein solches Implantat ermöglicht es, die erforderliche Menge an Zellen in den Nukleus zu injizieren, um das Bandscheibenfach bei einem einzigen Eingriff in der ursprünglichen Höhe zu restaurieren. Ein Druck innerhalb der Bandscheibe wird durch das Bandscheibenentlastungsimplantat deutlich verringert, sodaß es nicht zu einem Absterben der injizierten Zellen kommt. Da das Implantat zudem aus einem biokompatiblen, resorbierbaren Material hergestellt ist, wird der Druck auf die restaurierte Bandscheibe erst im Laufe der Zeit erhöht. Es bleibt daher genügend Zeit für eine ausreichende Stabilisierung der Bandscheibe mit den injizierten Zellen. Durch entsprechende Wahl des Materials sowie Formgebung des Implantats kann erreicht werden, daß eine Entlastung des Bandscheibenfachs im Verlauf von einigen Wochen sukzessive verringert wird, beispielsweise in einem Zeitraum von zwei Wochen bis 18 Monaten. Durch die Resorbierbarkeit des Implantats wird die Entlastung des Bandscheibenfachs nach und nach reduziert, wohingegen sie bei einem nichtresorbierbaren Implantat infolge des Entfernen des Implantats in einem Schritt vollständig ausgeschaltet wird. Ferner ist es von Vorteil, daß das Implantat mindestens teilweise, üblicherweise vollständig resorbierbar ist, so daß kein weiterer Eingriff erforderlich ist, um das Bandscheibenentlastungsimplantat wieder zu entfernen. Insbesondere das Entfernen eines internen Fixateurs zum Entlasten des Bandscheibenfachs kann zu einem unerwünschten Trauma führen. Des weiteren kann das Implantat bei entsprechender Formgebung auch durch einen minimalinvasiven Zugang in den Körper eingeführt werden, wodurch ein Operationsstrauma minimiert und damit eine postoperative Beeinträchtigung des Patienten verringert wird. Um das Implantat universell einsetzen zu können, insbesondere bei Patienten unterschiedlicher Größe, ist es günstig, dass ein Abstand zwischen den mindestens zwei Anlageelementen veränderbar ist. Dies ermöglicht es, das Implantat individuell an einen Patienten anzupassen und auch eine gewünschte Entlastung des Bandscheibenfachs gezielt vorzugeben. Damit die Anlageelemente einen eingestellten Abstand voneinander beibehalten, ist es vorteilhaft, dass die zwei Anlageelemente in einem bestimmten Abstand relativ zueinander festlegbar sind.

Günstig ist es, wenn mindestens eines der mindestens zwei Anlageelemente in Form einer Aufnahme mit einer Einführöffnung zum Einführen eines Dornfortsatzes in einer Richtung parallel oder quer zu einer vom Dornfortsatz definierten Vorzugsrichtung ausgebildet ist. Dadurch wird zum einen das Einbringen des Implantats in den Körper und das Ansetzen an den oder die Dornfortsätze vereinfacht. Zum anderen kann unter Umständen völlig auf zusätzliche Befestigungselemente zum Festlegen des Implantats verzichtet werden, was einerseits eine Operationszeit verringert und andererseits keine zusätzliche Verletzung eines Dornfortsatzes erforderlich macht. Zudem kann eine Bewegung eines Dornfortsatzes durch einen Anschlag, wie er beispielsweise durch eine gabelförmige Aufnahme definiert wird, in gewünschter Weise begrenzt werden.

Besonders einfach wird der Aufbau des Implantats, wenn zwei Aufnahmen vorgesehen sind, deren Einführöffnungen voneinander weg weisen. Beispielsweise könnte das Implantat in Form eines Doppel-T-Trägers ausgebildet sein, welcher zwei gabelförmige Aufnahmen zum Einführen von zwei Dornfortsätzen definiert. Des weiteren kann durch eine solche Ausgestaltung das Implantat besonders schlank und langgestreckt ausgebildet werden, was das Einführen des Implantats durch einen minimalinvasiven Zugang in den Körper vereinfacht. Außerdem wird durch die Ausgestaltung des Implantat ein minimaler Abstand der zwei benachbarten Dornfortsätze vorgegeben.

Vorzugsweise ist die Aufnahme nutförmig ausgebildet. Ein solches Implantat kann parallel zur nutförmigen Aufnahme an die Dornfortsätze herangeführt werden. Zudem ist es aber auch möglich, die Dornfortsätze quer zur Nutrichtung in die Aufnahme einzuführen.

Das Implantat wird besonders leicht und ist zudem sehr einfach herzustellen, wenn es ganz oder teilweise aus einem Kunststoff hergestellt ist. Ein weiterer Vorteil, das Implantat aus einem Kunststoff herzustellen, ist, daß sich das Implantat insbesondere dann, wenn der Kunststoff gummielastische Eigenschaften aufweist, optimal an eine anatomische Geometrie anpassen kann und auf diese Weise Traumata vermieden werden können.

Um eine Resorbierbarkeit des Implantats innerhalb eines gewünschten Zeitraums vorgeben zu können, ist es vorteilhaft, wenn der Kunststoff ein Polymer ist oder ein Polymer enthält. Infolge der Resorption können Polymerketten sukzessive abgebaut werden, wodurch die Stabilität des Implantats insgesamt im Laufe der Zeit in gewünschter Weise verringert wird.

Besonders körperverträglich ist das Implantat, wenn das Polymer Polylactid ist oder Polylactid enthält.

Ferner läßt sich insbesondere eine Resorbierbarkeit des Implantats besonders gut einstellen, wenn der Kunststoff eine dreidimensional vernetzte Gelatine ist oder eine dreidimensional vernetzte Gelatine enthält.

Um zu verhindern, daß sich das Implantat aus einer vorgegebenen Position relativ zu einem Dornfortsatz von diesem löst, ist es günstig, wenn das Implantat mindestens ein Sicherungselement umfaßt zum Sichern eines Dornfortsatzes an einem Anlageelement. Beispielsweise kann das Sicherungselement ein Befestigungselement sein, mit dem das Anlageelement an einem

Dornfortsatz festlegbar ist. Allerdings wäre es auch denkbar, ein Sicherungselements vorzusehen, welches die Bewegungsfreiheit des Implantats relativ zu einem Dornfortsatz nur bedingt einschränkt, beispielsweise durch Verringern der Zahl der Bewegungsfreiheitsgrade des Implantats relativ zu einem Dornfortsatz von zwei auf einen.

Damit ein Dornfortsatz nicht aus der Aufnahme wieder durch die Einführöffnung austreten kann, ist es vorteilhaft, wenn die Einführöffnung verschließbar oder verriegelbar ist.

Günstigerweise ist ein Verschlußelement zum Verschließen der Einführöffnung vorgesehen. Das Verschlußelement kann ein eigenes Bauteil oder aber auch einstückig mit dem übrigen Implantat ausgebildet sein.

Damit die Einführöffnung auf einfache Weise verschlossen werden kann, ist es vorteilhaft, wenn das Verschlußelement beweglich an der Aufnahme gelagert ist. Beispielsweise kann es so gelagert sein, daß es sich nicht in unbeabsichtigter Weise vom Implantat lösen kann, zum Beispiel über ein Filmscharnier.

Besonders einfach läßt sich die Einführöffnung verschließen, wenn das Verschlußelement verschwenkbar oder verschiebbar gelagert ist. Auf diese Weise kann insbesondere durch einfaches Verschwenken des Verschlußelements die Einführöffnung verschlossen werden, beispielsweise nach Einsetzen und Anlegen des Implantats an einen Dornfortsatz.

Gemäß einer bevorzugten Ausführungsform der Erfindung kann vorgesehen sein, daß das Verschlußelement in einer Verschlußstellung, in welcher die Einführöffnung verschlossen, ist an der Aufnahme verriegelbar oder verrastbar ist. So läßt sich auf einfache Weise verhindern, daß das Verschlußelement die Einführöffnung in unbeabsichtigter Weise freigibt.

Vorteilhafterweise ist der Abstand zwischen den beiden Anlageelementen in diskreten Schritten veränderbar. Dadurch wird zum einen der Aufbau des Implantats vereinfacht, beispielsweise durch Vorsehen von gegeneinander verschiebbaren Rastleisten oder Verzahnungen, zum anderen wird auch die Handhabbarkeit sowie die Stabilität des Implantats verbessert.

Grundsätzlich wäre es denkbar, das Implantat einstückig auszubilden. Günstig ist es jedoch, wenn das Implantat mindestens zwei, jeweils mindestens ein Anlageelement tragende Implantatteile umfaßt und wenn die zwei Implantatteile aneinander festlegbar sind. Dadurch kann beispielsweise eine relative Anordnung der Anlageelemente individuell und je nach Bedarf für jeden Patienten unterschiedlich festgelegt werden. Außerdem läßt sich so auch ein Abstand der Anlageelemente voneinander und damit eine Entlastung des Bandscheibenfachs in gewünschter Weise vorgeben.

Vorzugsweise sind die zwei Implantatteile in unterschiedlichen Positionen relativ zueinander festlegbar. Dies kann in Form sowohl diskreter als auch nicht diskreter Positionen möglich sein.

Damit das Implantat auf einfache Weise sicher an einem Dornfortsatz festgelegt werden kann, ist es günstig, wenn das Implantat mindestens ein Befestigungselement umfaßt zum Festlegen des mindestens einen Anlageelements an einem Dornfortsatz.,

Um die Stabilität des Implantats zu erhöhen und zudem sicherzustellen, daß sich das Implantat nicht von einem Dornfortsatz lösen kann, ist es vorteilhaft, wenn das Implantat für das mindestens eine Befestigungselement mindestens eine Befestigungselementaufnahme aufweist, daß das Befestigungselement in den Dornfortsatz eintreibbar oder an diesem festlegbar ist und daß das Befestigungselement in der Befestigungselementaufnahme gehalten ist. Beispielsweise könnte ein Knochenpin oder eine Knochenschraube durch eine eine Befestigungselementaufnahme bildende Bohrung des Implantats hindurchgeführt und in den Knochen eingetrieben werden. Alternativ könnte auch ein Faden durch eine Bohrung hindurchgeführt und um den Dornfortsatz gewickelt werden. Es wäre jedoch auch denkbar, das Befestigungselement in Form eines Dübels oder einer Niete auszubilden.

Um die Stabilität einer Verbindung zwischen dem Implantat und einem Dornfortsatz zu erhöhen, ist es günstig, wenn das mindestens eine Befestigungselement die Aufnahme quer durchsetzend an dieser festlegbar ist.

Vorteilhafterweise sind vier Befestigungselemente pro Anlageelement vorgesehen. Dadurch kann eine besonders sichere Verbindung zwischen dem Implantat und dem Dornfortsatz erreicht werden.

Gemäß einer bevorzugten Ausführungsform der Erfindung kann vorgesehen sein, daß das mindestens eine Befestigungselement ein Knochenpin, eine Knochenschraube oder ein Faden ist. Mit derartigen Befestigungselementen läßt sich das Implantat auf einfache Weise an einem Dornfortsatz festlegen. Das mindestens eine Befestigungselement kann zudem, wie das gesamte Implantat, aus einem resorbierbaren Material hergestellt sein, beispielsweise aus dem gleichen Material wie das übrige Implantat. Es wäre jedoch auch denkbar, Befestigungselemente vorzusehen, die zwar aus einem biokompatiblen, jedoch nicht resorbierbaren Material hergestellt sind.

Ferner ist es bei einem Verfahren der eingangs beschriebenen Art günstig, wenn das Implantat aus einem biokompatiblen, resorbierbaren Material hergestellt wird.

Mit dem Verfahren ist es möglich, ein Aufrichten des Bandscheibenfachs, beispielsweise durch Injektion eines hierfür erforderlichen Zellvolumens in den Nukleus der Bandscheibe, in einem einzigen chirurgischen Eingriff zu erreichen, ohne Gefahr laufen zu müssen, daß die injizierten Zellen aufgrund eines erhöhten Innendrucks im Nukleus absterben. Ferner gestattet einem die vorgeschlagene Vorgehensweise, ein Implantat auszuwählen, welches in einem bestimmten Zeitbereich seine Entlastungsfunktion nach und nach verringert.

Vorteilhaft ist es, wenn mindestens eines der mindestens zwei Anlageelemente in Form einer Aufnahme mit einer Einführöffnung zum Einführen eines Dornfortsatzes in einer Richtung parallel oder quer zu einer vom Dornfortsatz definierten Vorzugsrichtung ausgebildet wird. Dadurch kann der Eingriff wesentlich schnelle durchgeführt werden, da ein Dornfortsatz auf einfache Weise in die Aufnahme eingeführt werden kann.

Vorzugsweise werden zwei Aufnahmen vorgesehen, deren Einführöffnungen voneinander weg weisen. Dadurch kann insbesondere verhindert werden, daß in die Aufnahmen eingeführte Dornfortsätze in einem Minimalabstand voneinander gehalten werden.

Ein Dornfortsatz kann in die Aufnahme besonders einfach eingeführt werden, wenn diese nutförmig ausgebildet wird.

Besonders einfach läßt sich ein Implantat durch einen minimalinvasiven Zugang in einen menschlichen Körper einführen, wenn das Implantat ganz oder teilweise aus einem Kunststoff hergestellt wird, beispielsweise kann dieser Kunststoff eine gewisse Elastizität aufweisen, sodaß das Implantat beim Einführen in den Körper etwas zusammengedrückt werden kann.

Vorzugsweise wird ein Kunststoff verwendet, der ein Polymer ist oder ein Polymer enthält. Eine Resorptionszeit des Implantats kann dadurch in gewünschter Weise vorgegeben werden.

Besonders körperverträglich wird ein Implantat, wenn ein Polymer verwendet wird, das Polylactid ist oder Polylactid enthält.

Vorzugsweise wird ein Kunststoff verwendet, der eine dreidimensional vernetze Gelatine ist oder eine dreidimensional vernetzte Gelatine enthält. Die Verwendung von Gelatine als Material für die Herstellung des Implantats ermöglicht es, insbesondere durch Einstellung eines Vernetzungsgrads der Gelatine, eine gewünschte Resorptionszeit des Implantats und damit eine sukzessive Entlastung des Bandscheibenfachs in einem bestimmten Zeitraum vorzugeben.

Um zu verhindern, daß sich das Implantat in unerwünschter Weise von einem Dornfortsatz lösen kann, ist es günstig, wenn das Implantat mindestens ein Sicherungselement umfaßt und wenn mit dem mindestens einen Sicherungselement ein Anlageelement an einem Dornfortsatz gesichert wird. Beispielsweise kann als Sicherungselement ein bereits oben näher beschriebenes Befestigungselement verwendet werden. Alternativ wäre es auch denkbar, ein Verschlußelement für die Einführöffnung vorzusehen, wodurch verhindert wird, daß ein Dornfortsatz aus der Aufnahme austreten kann.

Um das Austreten eines Dornfortsatzes aus der Aufnahme zu verhindern, ist es vorteilhaft, wenn die Einführöffnung nach dem Einführen des Dornfortsatzes in die Aufnahme verschlossen wird.

Die Einführöffnung läßt sich besonders einfach verschließen, wenn sie mit einem Verschlußelement verschlossen wird. Ein speziell zum Verschließen der Einführöffnung vorgesehenes Verschlußelement hilft sicherzustellen, daß eine Dauer des Eingriffs minimiert wird.

Besonders einfach läßt sich die Einführöffnung mit dem Verschlußelement verschließen, wenn das Verschlußelement beweglich an der Aufnahme gelagert wird. Es muß dann beispielsweise nach Einführen des Dornfortsatzes in die Aufnahme von einem Operateur nur von einer Einführstellung, in der die Einführöffnung freigegeben ist, in eine Verschlußstellung, in der die Einführöffnung verschlossen ist, bewegt werden.

Besonders schnell und einfach läßt sich das Verschlußelement in die Verschlußstellung überführen, wenn das Verschlußelement verschwenkbar oder verschiebbar gelagert wird.

Damit eine sichere Verbindung zwischen dem Implantat und dem Dornfortsatz erreicht werden kann, ist es vorteilhaft, wenn das Verschlußelement in einer Verschlußstellung, in welcher die Einführöffnung verschlossen ist, an der Aufnahme verriegelt oder verrastet wird.

Günstigerweise wird ein Abstand zwischen den mindesten zwei Anlageelementen vor oder nach dem Einsetzen des Implantats in gewünschter Weise verändert. Auf diese Weise kann das Implantat individuell auf den jeweiligen Patienten abgestimmt werden, insbesondere auf Situationen, bei denen Abstände von Dornfortsätzen stark variieren.

Um zu verhindern, daß sich ein Abstand zwischen den zwei Anlageelementen nach Einsetzen in den menschlichen Körper und Abschluß des Eingriffs verändern kann, ist es günstig, wenn die zwei Anlageelemente vor oder nach dem Einsetzen des Implantats in einem bestimmten Abstand relativ zueinander festgelegt werden.

Für einen Operateur läßt sich ein Abstand zwischen den beiden Anlageelementen in vorteilhafter Weise ohne zusätzliche Werkzeuge durchführen, wenn zum Beispiel der Abstand zwischen den beiden Anlageelementen vor oder nach dem Einsetzen des Implantats in diskreten Schritten verändert wird. Beispielsweise kann dies über das Vorsehen relativ zueinander verschiebbarer Rastleisten oder korrespondierender Verzahnungen erreicht werden.

Um einen individuelle Anpassung des Implantats an einen Patienten zu ermöglichen, ist es günstig, wenn das Implantat mindesten zwei, jeweils mindestens ein Anlageelement tragende Implantatteile umfaßt und wenn die zwei Implantatteile vor oder nach dem Einsetzen des Implantats aneinander festgelegt werden. Damit kann beispielsweise ein Abstand der beiden Anlageelemente eingestellt und damit eine gewünschte Entlastung des Bandscheibenfachs vorgegeben werden.

Vorteilhafterweise sind die zwei Implantatteile in unterschiedlichen Positionen relativ zueinander festlegbar und werden in einer bestimmten Position aneinander festgelegt. Dadurch kann noch während der Operation das Implantat entsprechend den Erfordernissen aufgrund einer orthopädischen Situation vorbereitet werden.

Vorteilhaft ist es, wenn das Implantat mindestens ein Befestigungselement umfaßt und wenn mit dem mindestens einen Befestigungselement das mindestens eine Anlageelement an einem Dornfortsatz festgelegt wird. Das Implantat läßt sich so auf einfache und sichere Weise an einem Dornfortsatz sichern.

Günstig ist es, wenn das Implantat für das mindestens eine Befestigungselement mindestens eine Befestigungselementaufnahme aufweist und wenn das Befestigungselement in die Befestigungselementaufnahme eingeführt und in den Dornfortsatz eingetrieben oder an diesem festgelegt wird. Beispielsweise kann durch Einschlagen eines Knochenpins oder Einschrauben einer Knochenschraube und vorherigem Durchführen dieser Befestigungselemente durch eine entsprechende Befestigungselementaufnahme des Implantats, beispielsweise eine Bohrung, das Implantat sicher an einem Dornfortsatz gehalten werden. Alternativ kann auch mit einem durch eine Bohrung hindurchgeführten Faden, der um einen Dornfortsatz gewickelt und an diesem verknotet wird, das Implantat an einem Dornfortsatz festgelegt werden.

Gemäß einer bevorzugten Variante des Verfahrens kann vorgesehen sein, daß das mindestens eine Befestigungselement die Aufnahme quer durchsetzend an dieser festgelegt wird. Eine Verbindung zwischen dem Implantat und dem Dornfortsatz wird so besonders gut gesichert. Insbesondere kann auch vorgesehen sein, daß vor dem Einführen des Befestigungselements der Dornfortsatz ebenfalls mit einer entsprechenden Aufnahme für das Befestigungselement, beispielsweise einer oder mehrerer Bohrungen, versehen wird.

Um eine besonders gute Sicherung des Implantats an einem Dornfortsatz zu gewährleisten, werden vier Befestigungselemente pro Anlageelement verwendet.

Besonders einfach läßt sich das Verfahren durchführen und damit das Implantat an einem Dornfortsatz festlegen, wenn als Befestigungselement ein Knochenpin, eine Knochenschraube oder ein Faden verwendet wird und wenn das Anlageelement an einem Dornfortsatz festgepint, festgeschraubt oder festgenäht wird.

Gemäß einer weiteren bevorzugten Variante des Verfahrens kann vorgesehen sein, daß ein minimalinvasiver Zugang zum menschlichen oder tierischen Körper eröffnet wird, daß das Implantat durch den minimalinvasiven Zugang hindurch an die Dornfortsätze der Wirbel herangeführt wird und daß bei diesem Eingriff auch Knorpelzellen in einen Nukleus einer deformierten oder geschädigten Bandscheibe injiziert werden. Diese Vorgehensweise macht es möglich, daß mit nur einem chirurgischen Eingriff und gleichzeitiger Minimierung eines Operationstraumas das Bandscheibenfach in seiner ursprünglichen Höhe aufgerichtet und mit dem Implantat entlastet wird.

Vorzugsweise wird ein Implantat verwendet, daß in einem Zeitraum von drei Wochen bis sechs Monaten vollständig resorbiert wird. Auf diese Weise wird den injizierten Zellen ausreichend Zeit gegeben, um die Stabilität der Bandscheibe in gewünschter Weise wiederherzustellen.

Die nachfolgende Beschreibung bevorzugter Ausführungsformen der Erfindung dient im Zusammenhang mit der Zeichnung der näheren Erläuterung. Es zeigen:
- Figur 1:: ein an Dornfortsätzen einer Wirbelsäule festgelegtes erstes Beispiel eines Implantats;
- Figur 2:: eine vergrößerte Ansicht des Implantats aus Figur 1;
- Figur 3:: eine perspektivische Ansicht eines zweiten Beispiels eines Implantats; und
- Figur 4:: eine perspektivische Ansicht eines Ausführungsbeispiels eines erfindungsgemäßen Implantats;

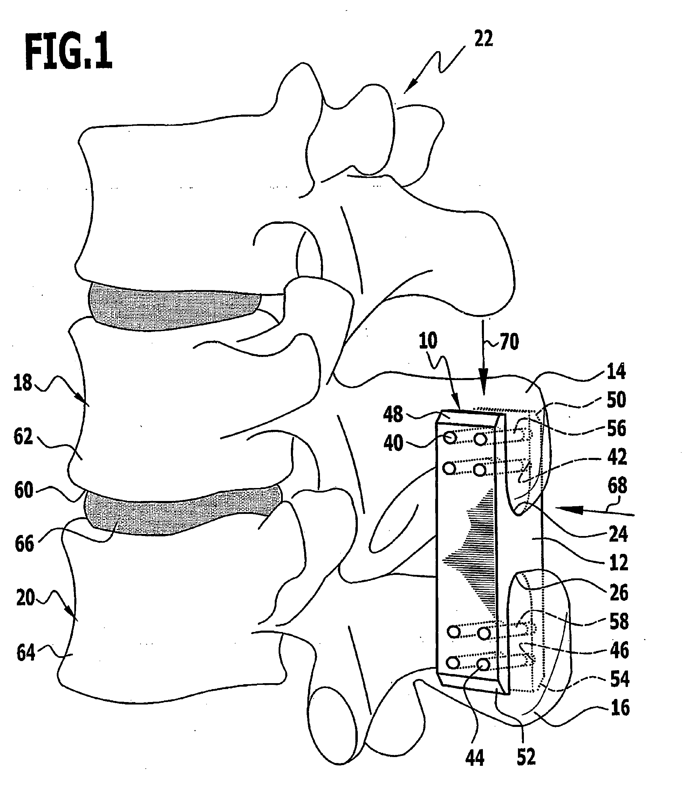

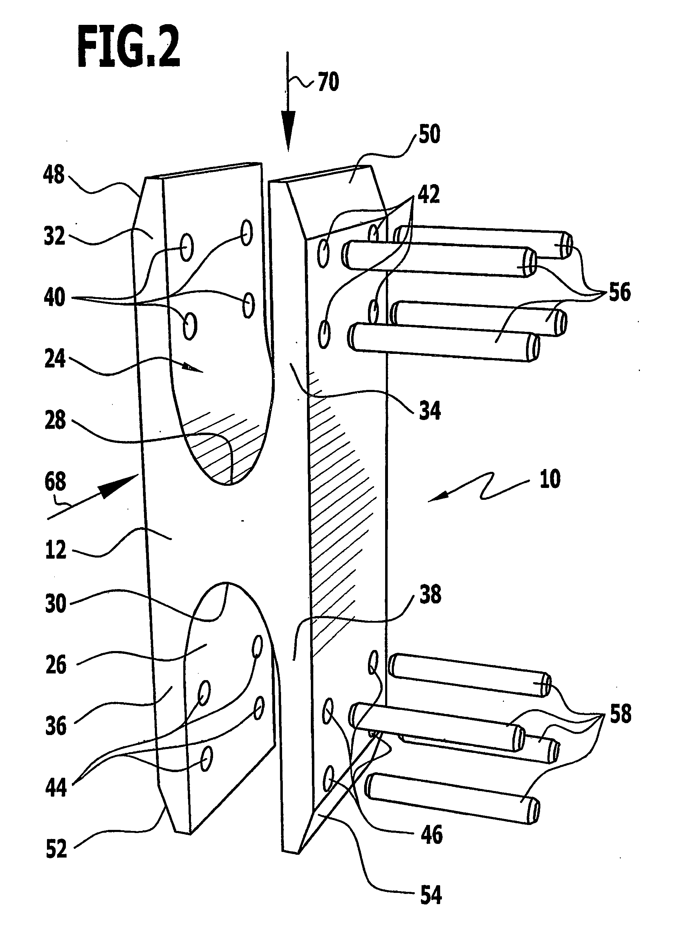

In den Figuren 1 und 2 ist ein insgesamt mit dem Bezugszeichen 10 versehenes, Bandscheibenentlastungsimplantat 10 dargestellt, welches nachfolgend der Einfachheit halber nur noch als Implantat bezeichnet wird.

Das Implantat 10 wird gebildet durch einen langgestreckten, im wesentlichen quaderförmigen Grundkörper 12, der einen in etwa quadratischen Querschnitt aufweist. Ausgehend von gegenüberliegenden Stirnflächen des Grundkörpers 12 sind zwei, voneinander weg weisende nutförmige Aufnahmen für einen Dornfortsatz 14 beziehungsweise 16 benachbarter Wirbel 18 und 22 einer Wirbelsäule 22 in Form nutförmiger Einschnitte 24 und 26 vorgesehen. Die Einschnitte 24 und 26 werden durch jeweils einen abgerundeten Nutboden 28 beziehungsweise 30 sowie jeweils zwei parallel zueinander, sich vom Nutboden 28 beziehungsweise 30 weg erstreckenden Wandabschnitten 32 und 34 beziehungsweise 36 und 38 begrenzt, die parallel zueinander ausgerichtet sind. In den Wandabschnitten 32 und 34 sind jeweils vier, in etwa ein Quadrat definierende und koaxial zueinander ausgerichtete Querbohrungen 40 und 42 vorgesehen. In analoger Weise sind in den Wandabschnitten 36 und 38 auch jeweils vier, zueinander koaxial ausgerichtete Querbohrungen 44 und 46 vorgesehen. Die Wandabschnitte 32, 34, 36 und 38 sind auf ihrer Außenseite ausgehend von ihrem freien Ende etwas abgeschrägt, so daß eine Wandstärke ausgehend vom jeweils freien Ende bis zu einer maximale Wandstärke hin kontinuierlich zunimmt und so geneigte Flächenabschnitte 48, 50, 52 und 54 gebildet werden.

Ebenso wie die Querbohrungen 40, 42, 44 und 46 nur optional oder in geringerer Anzahl oder auch nur an einem der beiden Einschnitte 24 und 26 in den Wandabschnitten 32, 34, 36 und 38 vorgesehen sein können, umfaßt das Implantat 10 auch nur optional insgesamt acht identische, im wesentlichen zylindrische Verriegelungsstifte, und zwar jeweils vier Verriegelungsstifte 56 und vier Verriegelungsstifte 58. Ein Außendurchmesser der Verriegelungsstifte 56 und 58 ist an einen Innendurchmesser der Querbohrungen 40 und 42 beziehungsweise 44 und 46 angepaßt, sodaß die Verriegelungsstifte 56 und 58 in die Querbohrungen 40 und 42 beziehungsweise 44 und 46 eingeschoben und in diesen klemmend gehalten werden können.

Sowohl der Grundkörper 12 als auch die Verriegelungsstifte 56 und 58 sind vorzugsweise aus einem resorbierbaren Kunststoff, beispielsweise einem Polymer wie Polylactid hergestellt.

Nachfolgend wird im Zusammenhang mit Figur 1 die operative Vorgehensweise zum Wiederaufrichten eines zwischen zwei Wirbelkörpern 62 und 64 gebildeten Bandscheibenfachs 60 näher erläutert.

Infolge eines Prolaps einer im Bandscheibenfach 60 zwischen den Wirbelkörpern 62 und 64 angeordneten Bandscheibe 66 kann Nukleusmaterial durch den Anulus der Bandscheibe 66 austreten. Dadurch verringert sich ein Abstand zwischen den Wirbelkörpern 62 und 64. Um diesen Höhenverlust auszugleichen, wird nach Eröffnen eines minimalinvasiven Zugangs ein zum Aufrichten der Bandscheibe 66 ausreichendes Zellvolumen in den Anulus der entlasteten Wirbelsäule 22 injiziert. Anschließend oder auch schon vor Injektion der Zellen wird durch den mininmalinvasiven Zugang das Implantat 10 eingeführt und von dorsal her kommend zwischen die Dornfortsätze 14 und 16 eingeschoben, das heißt in Richtung des Pfeils 68. Alternativ dazu wäre es auch denkbar, die Dornfortsätze 14 und 16 jeweils quer zu einer von diesen definierten Richtung, die im wesentlichen parallel zu einer vom Pfeil 68 vorgegebenen Richtung verläuft, in die Einschnitte 24 beziehungsweise 26 eingeschoben werden, also zum Beispiel der Dornfortsatz 14 in Richtung des Pfeils 70 in den Einschnitt 24.

Optional kann in einem nächsten Schritt, falls das Implantat Querbohrungen 40, 42, 44 und 46 in den Abschnitten 32, 34, 36 oder 38 aufweist, koaxial zu den Querbohrungen 40, 42, 44 und 46 Querbohrungen in den Dornfortsätzen 14 und 16 gesetzt werden, sodaß die Verriegelungsstifte 56 und 58 nicht nur durch die Querbohrungen 40, 42, 44 und 46 hindurchgeschoben werden können, sondern auch durch die nunmehr in den Dornfortsätzen 14 und 16 vorgesehenen Querbohrungen. Auf die beschriebene Weise kann das Implantat 10 an einem oder auch an beiden Dornfortsätzen 14 beziehungsweise 16 gesichert werden. Durch die Sicherung wird gewährleistet, daß sich das Implantat 10 nicht vom jeweils daran festgelegten Dornfortsatz 14 beziehungsweise 16 lösen kann. Ohne die beschriebene Sicherung mit den Verriegelungsstiften 56 und 58 wäre lediglich eine Bewegung der beiden Dornfortsätze 14 und 16 aufeinander zu begrenzt, nicht jedoch eine Bewegung der beiden Dornfortsätze 14 und 16 voneinander weg.

Die durch Zellinjektion wiederaufgerichtete Bandscheibe 66 wird durch das Implantat 10 entlastet, sodaß der Druck in der Bandscheibe 66 nicht so groß werden kann, daß die injizierten Zellen absterben.

Nach dem optionalen Festlegen des Implantats 10 in den Dornfortsätzen 14 und 16 kann der minimalinvasive Zugang wieder verschlossen werden. Selbstverständlich kann zur Durchführung des beschriebenen Eingriffs der Körper des Patienten auch durch einen größeren Zugang eröffnet werden.

Durch das Implantat 10 wird die Beweglichkeit des Bandscheibenfachs 60 begrenzt, bis das Implantat 10 so weit resorbiert ist, daß es durch aufgrund einer Bewegung der Wirbelsäule 22 auf das Implantat 10 wirkenden Kräften zerstört werden kann. Bis zu diesem Zeitpunkt der Resorption des Implantats 10 haben die injizierten Zellen die Möglichkeit, das Bandscheibenfach 60 mit belastungsfähigem Knorpelgewebe auszufüllen.

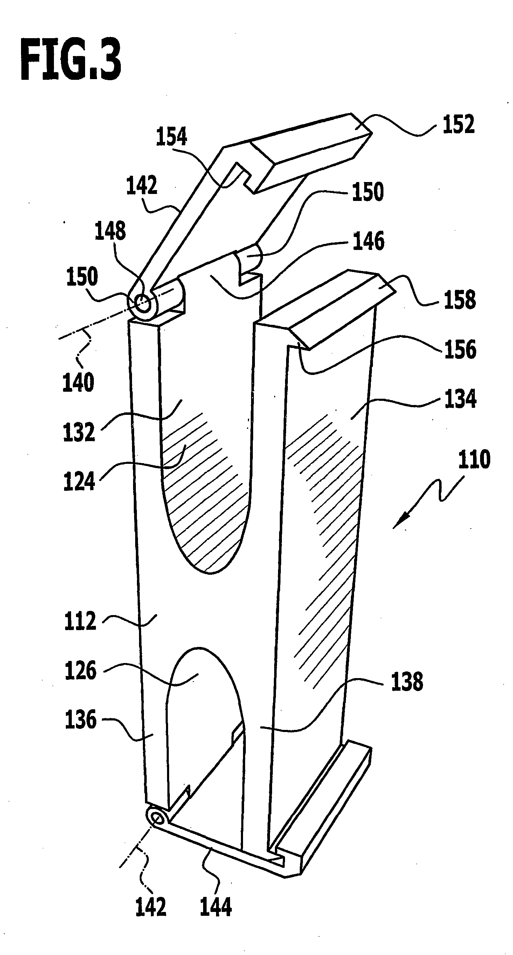

Im Zusammenhang mit Figur 3 wird nachfolgend ein zweites, insgesamt mit dem Bezugszeichen 110 versehenes Implantat näher beschrieben. Es weist einen analog zum Grundkörper 12 geformten Grundkörper 112 auf, der mit Einschnitten 124 und 126 versehen ist, die in ihrer Form den Einschnitten 24 und 26 entsprechen.

Die Wandabschnitte 132, 134 beziehungsweise 136 und 138 der Einschnitte 124 und 126 sind jedoch nicht mit Querbohrungen versehen. Dagegen sind an freien Enden der Wandabschnitte 132 und 136 jeweils um eine Schwenkachse 140 beziehungsweise 142, die parallel zueinander verlaufen, zwei Deckel 142 und 144 schwenkbar gelagert. Die Deckel 142 und 144 sind identisch ausgebildet, sodaß deren Lagerung nachfolgend nur in Verbindung mit dem Deckel 142 näher beschrieben wird.

Das freie Ende des Wandabschnitts 132 trägt einen parallel zur Schwenkachse 140 durchbohrten Lagerbock 146, durch den sich eine Lagerwelle 148 erstreckt. Beidseitig aus dem Lagerbock 146 vorstehende freie Enden der Lagerwelle 148 tauchen in beidseits an den Lagerbock 146 angrenzende Lagerösen 150 des Deckels 142 ein, sodaß der Deckel 142 um die von der Lagerwelle 148 definierte Schwenkachse relativ zum Grundkörper 112 verschwenkt werden kann. Ein freies Ende des Deckels 142 trägt einen Rastvorsprung 152, der eine in Richtung auf die Schwenkachse 140 hin weisende Rastnut 154 aufweist. Am Wandabschnitt 134 ist auf einer Außenseite, von der Schwenkachse 140 weg weisend, eine Rastnase 156 mit einer geneigten Aufgleitfläche 158 angeformt.

Wird das freie Ende des Deckels 142 in Richtung auf die Rastnase 156 hin bewegt, so gleitet zunächst eine Außenfläche des Rastvorsprungs 152 an der Aufgleitfläche 158 auf, wodurch der Deckel 142 etwas verformt wird. Wird der Deckel 142 weiter verschwenkt, dann schnappt die Rastnase 156 in die Rastnut 154 ein, wodurch der Einschnitt 124 stirnseitig verschlossen ist. In Figur 3 ist unten dargestellt, wie der Deckel 144 den Einschnitt 126 in der beschriebenen Weise verschließt.

Das Implantat 110 wird in ähnlicher Weise wie das Implantat 10 zwischen die Dornfortsätze 14 und 16 eingesetzt. Die Einschnitte 124 und 126 sind so dimensioniert, daß die Dornfortsätze 14 und 16 in diese eingeführt und durch stirnseitiges Verschließen der Einschnitte 124 und 126 in diesen gesichert werden können. Die Deckel 142 und 144 können entweder vor oder nach dem Einführen der Dornfortsätze 14 beziehungsweise 16 geschlossen werden.

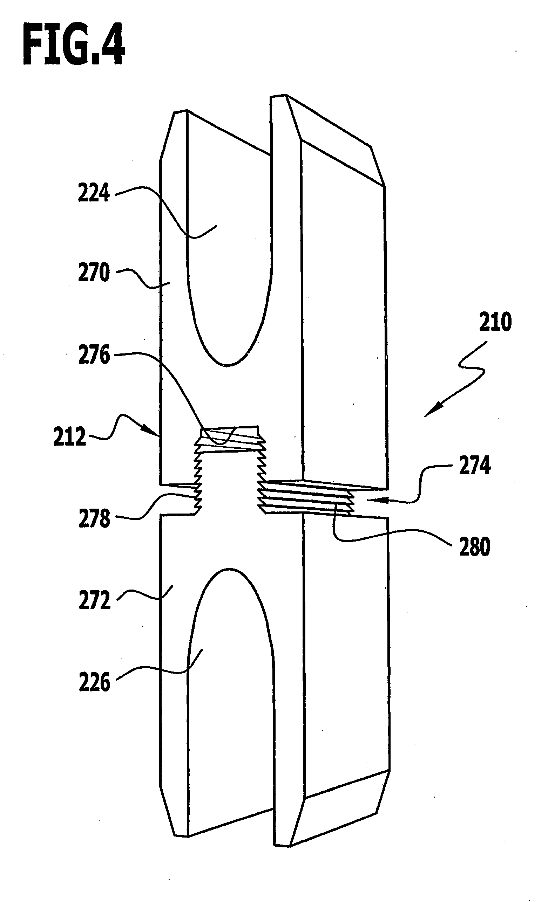

Ein erfindungsgemäßes Ausführungsbeispiel eines insgesamt mit dem Bezugszeichen 210 versehenen Implantats ist in Figur 4 dargestellt. Es entspricht in seiner Grundform im wesentlichen dem Implantat 10. Allerdings ist das Implantat 210 zweiteilig ausgebildet, das heißt der Grundkörper 212 ist in einen oberen Teil 270 und einen unteren Teil 272 geteilt. Der obere Teil 270 trägt einen dem Einschnitt 24 entsprechenden Einschnitt 224, der untere Teil 272 trägt einen dem Einschnitt 26 entsprechenden Einschnitt 226.

Zum Verbinden der beiden Teile 270 und 272 ist eine Rastverbindung 274 vorgesehen, die eine verzahnte Nut 276 und einen in die verzahnte Nut einschiebbaren Vorsprung 278 umfaßt. Die Nut 276 ist parallel verlaufend zum Einschnitt 224 am oberen Teil 270 vorgesehen. Der Vorsprung 278 dagegen steht am unteren Teil 272 von dem Einschnitt 226 weg weisend ab. Aneinander anliegende Seitenflächen der Nut 276 sowie des Vorsprungs 278 sind mit zueinander korrespondierenden Zahnreihen 280 versehen, die eine Bewegung der beiden Teile 270 und 272 aufeinander zu infolge eines gegenseitigen Aufgleitens einzelner Zähne der Zahnreihen 280 gestatten, jedoch nicht eine Bewegung der Teile 270 und 272 voneinander weg.

Ein Abstand der Einschnitte 224 und 226 kann vor dem Einführen des Implantats 210 in den Körper oder noch nach dem Einführen verändert werden. Bei dem beschriebenen Ausführungsbeispiel ist es nur schwer möglich, den Abstand zwischen den Einschnitten 224 und 226 zu vergrößern. Daher wird das Implantat 210 vorzugsweise mit einem maximal möglichen Abstand zwischen den Einschnitten 224 und 226 in den Körper des Patienten eingeführt und bei Bedarf der Abstand durch Aufeinanderzubewegung der beiden Teile 270 und 272 verringert.

Optional können am Implantat 210 eine oder mehrere Querbohrungen wie beim Implantat 10 oder ein oder zwei Deckel wie beim Implantat 110 vorgesehen sein.

## Patentansprüche

1. Bandscheibenentlastungsimplantat (210) zum Aufrichten und Entlasten eines Bandscheibenfachs (60) einer menschlichen oder tierischen Wirbelsäule (22), mit mindestens zwei Anlageelementen (224, 226) für jeweils einen Dornfortsatz (14, 16) zum Anlegen und/oder Festlegen des Implantats (210) an einem oder zwei Dornfortsätzen (14, 16) benachbarter Wirbel (18, 20) der Wirbelsäule (22), wobei das Implantat (210) aus einem biokompatiblen, resorbierbaren Material hergestellt ist, **dadurch gekennzeichnet, daß** ein Abstand zwischen den mindestens zwei Anlageelementen (224, 226) veränderbar ist und daß die zwei Anlageelemente (224, 226) in einem bestimmten Abstand relativ zueinander festlegbar sind.

2. Implantat nach Anspruch 1, **dadurch gekennzeichnet, daß** mindestens eines der mindestens zwei Anlageelemente (224, 226) in Form einer Aufnahme mit einer Einführöffnung zum Einführen eines Dornfortsatzes (14, 16) in einer Richtung parallel (68) oder quer (70) zu einer vom Dornfortsatz (14, 16) definierten Vorzugsrichtung ausgebildet ist.

3. Implantat nach Anspruch 2, **dadurch gekennzeichnet, daß** zwei Aufnahmen (224, 226) vorgesehen sind, deren Einführöffnungen voneinander weg weisen.

4. Implantat nach einem der Ansprüche 2 oder 3, **dadurch gekennzeichnet, daß** die Aufnahme (224, 226) nutförmig ausgebildet ist.

5. Implantat nach einem der voranstehenden Ansprüche, **dadurch gekennzeichnet, daß** das Implantat (210) ganz oder teilweise aus einem Kunststoff hergestellt ist.

6. Implantat nach Anspruch 5, **dadurch gekennzeichnet, daß** der Kunststoff ein Polymer ist oder ein Polymer enthält.

7. Implantat nach Anspruch 6, **dadurch gekennzeichnet, daß** das Polymer Polylactid ist oder Polylactid enthält.

8. Implantat nach einem der Ansprüche 6 oder 7, **dadurch gekennzeichnet,**
**daß** der Kunststoff eine dreidimensional vernetzte Gelatine ist oder eine dreidimensional vernetzte Gelatine enthält.

9. Implantat nach einem der voranstehenden Ansprüche, **dadurch gekennzeichnet, daß** das Implantat (210) mindestens ein Sicherungselement (56, 58) umfaßt zum Sichern eines Dornfortsatzes (14, 16) an einem Anlageelement (24, 26).

10. Implantat nach einem der Ansprüche 2 bis 9, **dadurch gekennzeichnet, daß** die Einführöffnung verschließbar ist.

11. Implantat nach einem der Ansprüche 2 bis 10, **dadurch gekennzeichnet, daß** ein Verschlußelement (142, 144) zum Verschließen der Einführöffnung vorgesehen ist.

12. Implantat nach Anspruch 11, **dadurch gekennzeichnet, daß** das Verschlußelement (142, 144) beweglich an der Aufnahme (124, 126) gelagert ist.

13. Implantat nach Anspruch 12, **dadurch gekennzeichnet, daß** das Verschlußelement (142, 144) verschwenkbar oder verschiebbar gelagert ist.

14. Implantat nach einem der Ansprüche 11 bis 13, **dadurch gekennzeichnet, daß** das Verschlußelement (142, 144) in einer Verschlußstellung, in welcher die Einführöffnung verschlossen ist, an der Aufnahme (124, 126) verriegelbar oder verrastbar ist.

15. Implantat nach einem der voranstehenden Ansprüche, **dadurch gekennzeichnet, daß** der Abstand zwischen den beiden Anlageelementen (224, 226) in diskreten Schritten veränderbar ist.

16. Implantat nach einem der voranstehenden Ansprüche, **dadurch gekennzeichnet, daß** das Implantat (210) mindestens zwei, jeweils mindestens ein Anlageelement (224; 226) tragende Implantatteile (270, 272) umfaßt und daß die zwei Implantatteile (270, 272) aneinander festlegbar sind.

17. Implantat nach Anspruch 16, **dadurch gekennzeichnet, daß** die zwei Implantatteile in unterschiedlichen Positionen relativ zueinander festlegbar sind.

18. Implantat nach einem der voranstehenden Ansprüche, **dadurch gekennzeichnet, daß** das Implantat (210) mindestens ein Befestigungselement (56, 58) umfaßt zum Festlegen des mindestens einen Anlageelements (24, 26) an einem Dornfortsatz (14, 16).

19. Implantat nach Anspruch 18, **dadurch gekennzeichnet, daß** das Implantat (210) für das mindestens eine Befestigungselement (56, 58) mindestens eine Befestigungselementaufnahme (40, 42, 44, 46) aufweist, daß das Befestigungselement (56, 58) in den Dornfortsatz (14, 16) eintreibbar oder an diesem festlegbar ist und daß das Befestigungselement (56, 58) in der Befestigungselementaufnahme (40, 42, 44, 46) gehalten ist.

20. Implantat nach einem der Ansprüche 18 oder 19, **dadurch gekennzeichnet, daß** das mindestens eine Befestigungselement (56, 58) die Aufnahme (24, 26) quer durchsetzend an dieser festlegbar ist.

21. Implantat nach einem der Ansprüche 18 bis 20, **dadurch gekennzeichnet, daß** vier Befestigungselemente (56, 68) pro Anlageelement (24, 26) vorgesehen sind.

22. Vorrichtung nach einem der Ansprüche 18 bis 21, **dadurch gekennzeichnet, daß** das mindestens eine Befestigungselement (56, 68) ein Knochenpin, eine Knochenschraube oder ein Faden ist.

## Claims

1. Implant (210) for alleviating pressure on intervertebral disks, for restoring the height of and alleviating pressure on an intervertebral space (60) of a human or animal spinal column (22), comprising at least two bearing elements (224, 226) for a spinous process (14, 16) each for abutting and/or securing the implant (210) on one or two spinous processes (14, 16) of adjacent vertebra (18, 20) of the spinal column (22), wherein the implant (210) is produced from a biocompatible, resorbable material, **characterized in that** a distance between the at least two bearing elements (224, 226) is variable and **in that** the two bearing elements (224, 226) are securable relative to one another at a predetermined distance.

2. Implant in accordance with claim 1, **characterized in that** at least one of the at least two bearing elements (224, 226) is designed in the form of a receptacle with an insertion opening for the insertion of a spinous process (14, 16) in a direction parallel (68) or transverse (70) to a preferred direction defined by the spinous process (14, 16).

3. Implant in accordance with claim 2, **characterized in that** two receptacles (224, 226) are provided, the insertion openings thereof pointing away from one another.

4. Implant in accordance with claim 2 or 3, **characterized in that** the receptacle (224, 226) is of a groove-like design.

5. Implant in accordance with any one of the preceding claims, **characterized in that** the implant (210) is produced entirely or partially from a plastic material.

6. Implant in accordance with claim 5, **characterized in that** the plastic material is a polymer or contains a polymer.

7. Implant in accordance with claim 6, **characterized in that** the polymer is polylactide or contains polylactide.

8. Implant in accordance with claim 6 or 7, **characterized in that** the plastic material is a gelatin cross-linked three dimensionally or contains a gelatin cross-linked three dimensionally.

9. Implant in accordance with any one of the preceding claims, **characterized in that** the implant (210) comprises at least one securing element (56, 58) for securing a spinous process (14, 16) to a bearing element (24, 26).

10. Implant in accordance with any one of claims 2 to 9, **characterized in that** the insertion opening is closable.

11. Implant in accordance with any one of claims 2 to 10, **characterized in that** a closure element (142, 144) for closing the insertion opening is provided.

12. Implant in accordance with claim 11, **characterized in that** the closure element (142, 144) is mounted on the receptacle (124, 126) so as to be movable.

13. Implant in accordance with claim 12, **characterized in that** the closure element (142, 144) is pivotally or displaceably mounted.

14. Implant in accordance with any one of claims 11 to 13, **characterized in that** the closure element (142, 144) is lockable or connectable in a snap-in manner to the receptacle (124, 126) in a closure position, the insertion opening being closed in said position.

15. Implant in accordance with any one of the preceding claims, **characterized in that** the distance between the two bearing elements (224, 226) is variable in discrete steps.

16. Implant in accordance with any one of the preceding claims, **characterized in that** the implant (210) comprises at least two implant parts (270, 272) each having at least one bearing element (224, 226) and **in that** the two implant parts (270, 272) are securable to one another.

17. Implant in accordance with claim 16, **characterized in that** the two implant parts are securable relative to one another in different positions.

18. Implant in accordance with any one of the preceding claims, **characterized in that** the implant (210) comprises at least one fixing element (56, 58) for securing the at least one bearing element (24, 26) to a spinous process (14, 16).

19. Implant in accordance with claim 18, **characterized in that** the implant (210) has at least one fixing element receptacle (40, 42, 44, 46) for the at least one fixing element (56, 58), **in that** the fixing element (56, 58) is adapted to be driven into the spinous process (14, 16) or secured to it and **in that** the fixing element (56, 58) is held in the fixing element receptacle (40, 42, 44, 46).

20. Implant in accordance with claim 18 or 19, **characterized in that** the at least one fixing element (56, 68) is securable to the receptacle (24, 26) passing transversely through it.

21. Implant in accordance with any one of claims 18 to 20, **characterized in that** four fixing elements (56, 58) are provided per bearing element (24, 26).

22. Implant in accordance with any one of claims 18 to 21, **characterized in that** the at least one fixing element (56, 58) is a bone pin, a bone screw or a thread.

## Revendications

1. Implant de délestage intervertébral (210) pour redresser et délester un espace de disque intervertébral (60) d'une colonne vertébrale (22) humaine ou animale, comprenant au moins deux éléments d'appui (224, 226) destinés chacun respectivement à une apophyse épineuse (14, 16) pour appliquer et/ou fixer l'implant (210) à l'un ou à deux apophyses épineuses (14, 16) de vertèbres voisines (18, 20) de la colonne vertébrale (22), l'implant (210) étant fabriqué en un matériau biocompatible et résorbable, **caractérisé en ce qu'**une distance d'espacement entre lesdits au moins deux éléments d'appui (224, 226) peut être modifiée, et **en ce que** les deux éléments d'appui (224, 226) peuvent être fixés l'un par rapport à l'autre selon une distance d'espacement réciproque déterminée.

2. Implant selon la revendication 1, **caractérisé en ce qu'**au moins l'un desdits au moins deux éléments d'appui (224, 226) est conçu et réalisé sous forme de logement d'accueil avec une ouverture d'introduction pour introduire une apophyse épineuse (14, 16) dans une direction parallèle (68) ou transversale (70) par rapport à une direction préférentielle définie par l'apophyse épineuse (14, 16).

3. Implant selon la revendication 2, **caractérisé en ce que** sont prévus deux logements d'accueil (224, 226) dont les ouvertures d'introduction sont orientées de manière mutuellement opposée.

4. Implant selon l'une des revendications 2 ou 3, **caractérisé en ce que** le logement d'accueil (224, 226) est réalisé sous forme de rainure.

5. Implant selon l'une des revendications précédentes, **caractérisé en ce que** l'implant (210) est fabriqué en totalité ou en partie en une matière plastique.

6. Implant selon la revendication 5, **caractérisé en ce que** la matière plastique est un polymère ou renferme un polymère.

7. Implant selon la revendication 6, **caractérisé en ce que** le polymère est un polylactide ou renferme du polylactide.

8. Implant selon l'une des revendications 6 ou 7, **caractérisé en ce que** la matière plastique est une gélatine réticulée en trois dimensions ou renferme de la gélatine réticulée en trois dimensions.

9. Implant selon l'une des revendications précédentes, **caractérisé en ce que** l'implant (210) comprend au moins un élément d'arrêt de sécurité (56, 58) pour bloquer une apophyse épineuse (14, 16) dans un élément d'appui (24, 26).

10. Implant selon l'une des revendications 2 à 9, **caractérisé en ce que** l'ouverture d'introduction peut être fermée.

11. Implant selon l'une des revendications 2 à 10, **caractérisé en ce qu'**il est prévu un élément de fermeture (142, 144) pour fermer l'ouverture d'introduction.

12. Implant selon la revendication 11, **caractérisé en ce que** l'élément de fermeture (142, 144) est monté mobile au niveau du logement d'accueil (124, 126).

13. Implant selon la revendication 12, **caractérisé en ce que** l'élément de fermeture (142, 144) est monté de manière à pouvoir pivoter ou coulisser.

14. Implant selon l'une des revendications 11 à 13, **caractérisé en ce que** l'élément de fermeture (142, 144) peut, dans une position de fermeture dans laquelle l'ouverture d'introduction est fermée, être verrouillé au logement d'accueil (124, 126) ou y être encliqueté.

15. Implant selon l'une des revendications précédentes, **caractérisé en ce que** la distance d'espacement entre les deux éléments d'appui (224, 226) peut être modifiée par paliers discrets.

16. Implant selon l'une des revendications précédentes, **caractérisé en ce que** l'implant (210) comprend au moins deux parties d'implant (270, 272) portant chacune respectivement au moins un élément d'appui (224, 226), et **en ce que** les deux parties d'implants (270, 272) peuvent être fixées l'une par rapport à l'autre.

17. Implant selon la revendication 16, **caractérisé en ce que** les deux parties d'implant peuvent être fixées l'une par rapport à l'autre dans différentes positions.

18. Implant selon l'une des revendications précédentes, **caractérisé en ce que** l'implant (210) comprend au moins un élément de fixation (56, 58) pour fixer ledit au moins un élément d'appui (24, 26) à une apophyse épineuse (14, 16).

19. Implant selon la revendication 18, **caractérisé en ce que** l'implant (210) présente, pour ledit au moins un élément de fixation (56, 58), au moins un logement d'accueil d'élément de fixation (40, 42, 44, 46), **en ce que** l'élément de fixation (56, 58) peut être enfoncé dans l'apophyse épineuse (14, 16) ou y être fixé, et **en ce que** l'élément de fixation (56, 58) est maintenu dans le logement d'accueil d'élément de fixation (40, 42, 44, 46).

20. Implant selon l'une des revendications 18 ou 19, **caractérisé en ce que** ledit au moins un élément de fixation (56, 58) traverse transversalement le logement d'accueil (24, 26) en pouvant y être fixé.

21. Implant selon l'une des revendications 18 à 20, **caractérisé en ce que** sont prévus quatre éléments de fixation (56, 58) par élément d'appui (24, 26).

22. Implant selon l'une des revendications 18 à 21, **caractérisé en ce que** ledit au moins un élément de fixation (56, 58) est une broche à os, une vis à os ou un fil.
